# EUROPÄISCHE PATENTSCHRIFT

(11) **EP 0 912 209 B1**
(45) Veröffentlichungstag und Bekanntmachung des Hinweises auf die Patenterteilung: **12.04.2000**
(21) Anmeldenummer: 97929272.9
(22) Anmeldetag: 24.06.1997
(51) Int. Cl.: A61M 5/32, A61M 5/28

(54) **INJEKTIONSSPRITZENKOPF MIT ORIGINALITÄTSVERSCHLUSS**
SYRINGE HEAD WITH WARRANTY SEAL
TETE DE SERINGUE D'INJECTION A FERMETURE ANTI-REUTILISATION

(30) Priorität: 25.06.1996 AT 112296
(43) Veröffentlichungstag der Anmeldung: 06.05.1999
(73) Patentinhaber: Pharma-Consult Ges.m.b.H., 2203 Grossebersdorf (AT)
(72) Erfinder: MEYER, Philippe, CH-8001 Zürich (CH)
(74) Vertreter: Holzer, Walter, Dipl.-Ing.
(86) Internationale Anmeldenummer: EP9703296
(87) Internationale Veröffentlichungsnummer: WO9749444

(56) Entgegenhaltungen:
- WO-A-86/03126
- WO-A-94/22511
- US-A- 3 333 682
- US-A- 4 820 275
- US-A- 5 250 037

## Beschreibung

Die Erfindung betrifft einen Injektionsspritzenkopf mit Originalitätsverschluß für einen Spritzenzylinder, der einen Zylinderhals aufweist, welcher mit einem axialen Flüssigkeitsaustrittskanal und einem Umfangswulst zur Verankerung des Injektionsspritzenkopfes versehen ist, mit einer Injektionskanüle, die in einem Kanülenträger montiert ist, aus dem sie an beiden axialen Enden herausragt und der in einer Kanülenträgerführung relativ zu dieser bewegbar angeordnet ist, und einer Kanülenschutzkappe, an die über eine Sollbruchstelle ein die Kanülenträgerführung festhaltender, am Umfangswulst des Zylinderhalses unter elastischer Verformung festlegbarer Verankerungsteil mit einer Dichtungsscheibe anschließt, wobei die Kanülenschutzkappe am Innenumfang in Axialrichtung verlaufende Rippen aufweist, welche mit entsprechenden Rippen des Kanülenträgers für einen Drehantrieb desselben in Eingriff stehen.

Ein derartiger Injektionsspritzenkopf ist zum Verschließen sogenannter Fertigspritzen bestimmt, d.h. von Spritzen, die mit einem flüssigen Arzneimittel gefüllt und mit einer Injektionskanüle versehen steril abgepackt und gebrauchsfertig angeboten werden. Es ist wesentlich, daß diese Fertigspritzen mit einem Originalitätsverschluß versehen sind, der es gestattet, zu erkennen, ob die Spritze noch mit der Originalfüllung gefüllt und steril ist. Der Injektionsspritzenkopf sollte die Kanüle ausreichend schützen, damit es bei der Endmontage des Spritzenkopfes am Spritzenzylinder zu keiner Beschädigung oder Verunreinigung der Kanüle kommen kann. Ferner ist für die Massenfertigung ein einfacher Aufbau der Injektionsspritze erforderlich.

Bei einem Injektionsspritzenkopf der einleitend angegebenen Art ergibt sich ferner das Problem, daß bei einem unbeabsichtigten Verdrehen der Schutzkappe gegenüber dem Verankerungsteil in einem falschen Drehsinn zwar die Sollbruchstelle durchbrochen wird, die Kanüle jedoch nicht durch die Dichtungsscheibe hindurch und in Verbindung mit dem im Spritzenzylinder enthaltenen Medikament getrieben wird.

Die Erfindung zielt darauf ab, einen Injektionsspritzenkopf zu schaffen, der in Verbindung mit herkömmlichen Spritzenzylindern eine Fertigspritze ergibt, die nicht nur einfach und unter sterilen Bedingungen aufgebaut sowie mit einem Originalitätsverschluß versehen werden kann, sondern bei der zugleich Maßnahmen getroffen sind, um Bedienungsfehler mit den erörterten nachteiligen Folgen zu vermeiden.

Gegenstand der Erfindung ist ein Injektionsspritzenkopf mit Originalitätsverschluß für einen Spritzenzylinder, der einen Zylinderhals aufweist, welcher mit einem axialen Flüssigkeitsaustrittskanal und einem Umfangswulst zur Verankerung des Injektionsspritzenkopfes versehen ist, mit einer Injektionskanüle, die in einem Kanülenträger montiert ist, aus dem sie an beiden axialen Enden herausragt und der in einer Kanülenträgerführung relativ zu dieser bewegbar angeordnet ist, und einer Kanülenschutzkappe, an die über eine Sollbruchstelle ein die Kanülenträgerführung festhaltender, am Umfangswulst des Zylinderhalses unter elastischer Verformung festlegbarer Verankerungsteil mit einer Dichtungsscheibe anschließt, wobei die Kanülenschutzkappe am Innenumfang in Axialrichtung verlaufende Rippen aufweist, welche mit entsprechenden Rippen des Kanülenträgers für einen Drehantrieb desselben in Eingriff stehen, wobei am Umfang des Kanülenträgers zwei axialsymmetrisch, mit vorbestimmter Steigung im wesentlichen V-förmig verlaufende und an ihrem der Dichtungsscheibe zugekehrten Ende ineinandermündende Führungsnuten für einen im Inneren der Kanülenträgerführung vorgesehenen Führungszapfen ausgebildet sind, der im unbetätigten Zustand des Injektionsspritzenkopfes in den der Dichtungsscheibe zugekehrten Mündungsbereich der Führungsnuten eingreift und bei einer Drehung der Kanülenschutzkappe in jeder der beiden Drehrichtungen unter Zerstörung der Sollbruchstelle relativ zu einer der beiden Führungsnuten gleitet, so daß der Kanülenträger gedreht und mit der Kanüle relativ zur Dichtungsscheibe bewegt wird, wobei an den der Dichtungsscheibe zugekehrten Mündungsbereich der Führungsnuten eine nach unten offene axiale Einführnut für den Führungszapfen anschließt, die sich in radialer Richtung gegen ihr Eintrittsende zu erweitert, wobei das der Dichtungsscheibe zugekehrte Ende der Injektionskanüle geschlossen und mit einer seitlichen Öffnung versehen ist, durch welche das Medikament nach dem Aktivieren des Spritzenkopfes in die Kanüle eintreten kann, und wobei die Dichtungsscheibe vorgelocht ist und die Injektionskanüle das Loch im unbetätigten Zustand des Injektionsspritzenkopfes verschließt.

Der erfindungsgemäß aufgebaute Injektionsspritzenkopf besteht lediglich aus vier Montageteilen, u.zw. der Schutzkappe mit Verankerungsteil, der Dichtungsscheibe, der Kanülenträgerführung und dem Kanülenträger mit Kanüle (geklebt oder HF-verschweißt), und ist daher einfach herzustellen und zusammenzubauen. Die über eine Sollbruchstelle mit dem Verankerungsteil verbundene Schutzkappe gewährleistet die Originalitätsverschlußfunktion. Die Kanüle ist im Spritzenkopf geschützt integriert, und in der Endmontage wird der gesamte Spritzenkopf mit allen vier Montageteilen als eine Einheit auf den Spritzenzylinder aufgesetzt, so daß es zu keinerlei Beschädigungen oder Verunreinigungen der Injektionskanüle kommen kann. Nach dem Aufsetzen des Spritzenkopfes kann der Spritzenzylinder befüllt und mit einer entsprechenden Kolbeneinheit abdichtend bestückt werden.

Die Aktivierung der Spritze erfolgt durch Verdrehen der Schutzkappe, wodurch die Sollbruchstelle reißt und der Kanülenträger in die Kanülenträgerführung hineingedreht wird, u.zw. mit beliebiger Drehrichtung. Dabei bewegt sich die Kanüle relativ zur Dichtungsscheibe, wodurch der Eintritt des Medikamentes in die Kanüle ermöglicht wird. Die Schutzkappe wird dann in axialer Richtung abgezogen und die Spritze ist zur Injektion bereit.

Die erfindungsgemäßen Führungsnuten am Umfang der Kanülenträgerführung gewährleisten, daß die Schutzkappe aus ihrer unbetätigten Stellung, d.h. bei noch unversehrter Sollbruchstelle, zur Betätigung des Spritzenkopfes in beiden Drehrichtungen gedreht werden kann, wodurch ein Eindrehen des Kanülenträgers in die Kanülenträgerführung bewirkt wird. Eine versehentliche Verdrehung der Schutzkappe unter Auftrennen der Sollbruchstelle, ohne daß die Injektionskanüle in die Wirkstellung gebracht würde, ist dadurch ausgeschlossen. Zugleich wird durch die erfindungsgemäße Ausbildung der Führungsnut der Zusammenbau erleichtert.

Es sei erwähnt, daß aus der US 5 250 037 ein Injektionsspritzenkopf bekannt ist, bei welchem am Umfang des Zylinderhalses im wesentlichen V-förmig verlaufende Führungsnuten für einen im Inneren der Kanülenschutzkappe vorgesehenen Führungszapfen ausgebildet sind. Durch diese Ausbildung wird erreicht, daß bei Betätigung der Schutzkappe in jeder der beiden Drehrichtungen der Kanülenträger gegen den Zylinderhals die Dichtungsscheibe durchstößt. Die bekannte Konstruktion hat jedoch den Nachteil, daß zunächst bedingt durch die Ausbildung der Schutzkappe ein keimfreier Verschluß nicht gegeben ist, wie dies bei der Erfindung durch das Aufschnappen der Schutzkappe auf den Umfangswulst des Zylinderhalses unter elastischer Verformung und die definierte Sollbruchstelle der Fall ist. Ferner ist bei der Entgegenhaltung kein kraft- und formschlüssiger Antrieb des Kanülenträgers vorhanden, wie dies erfindungsgemäß durch den gegenseitigen Rippeneingriff zwischen Schutzkappe und Kanülenträger der Fall ist. Bei der erfindungsgemäßen Konstruktion wird außerdem durch die axiale Einführnut für den Führungszapfen der Zusammenbau erleichtert und es wird ferner durch die vorgelochte, im unbetätigten Zustand durch die Kanüle verschlossene Dichtungsscheibe einerseits die Betätigung erleichtert, anderseits die Gefahr eines Eindringens von Dichtungsscheibenmaterial in die Kanüle vermieden.

Die erfindungsgemäße Ausbildung bietet somit eine optimale Keimfreiheit bei verläßlicher Betätigung in beiden Drehrichtungen.

Nach einem weiteren Merkmal der Erfindung ist die Steigung der Führungsnuten so getroffen, daß etwa eine 1/4 Umdrehung des Kanülenträgers einen Weg der Kanüle relativ zur Dichtungsscheibe von etwa 3 mm bewirkt. Durch diesen Weg ist gesichert, daß nach ca. einer 1/4 Umdrehung bei gleichzeitiger Zerstörung des Originalitätsverschlusses der Kanülenschutzkappe die Kanüle so weit durch die Dichtungsscheibe gedrungen ist, daß die seitliche Eintrittsöffnung der Kanüle frei wird und das Medikament nach dem Abnehmen der bereits abgedrehten Kanülenschutzkappe ungehindert ausgespritzt werden kann.

Die Verankerung des Injektionsspritzenkopfes am Spritzenzylinder kann durch Aufpressen oder eine Rastverbindung erzielt werden. Bevorzugt wird die letztere Möglichkeit angewandt, in welchem Fall der Verankerungsteil aus elastischem Material gefertigt und auf den Umfangswulst des Zylinderhalses aufgeschnappt wird.

Ein weiteres bevorzugtes Merkmal der Erfindung besteht darin, daß der Verankerungsteil innenseitig konisch ausgebildet ist, wobei die Kanülenträgerführung in den Verankerungsteil von dessen distalem Ende her eingepreßt wird. Der Preßsitz erleichtert und beschleunigt die Montage der Fertigspritze.

Die Montage der Fertigspritze kann weiter vereinfacht werden, wenn gemäß einem bevorzugten Merkmal der Erfindung an der Innenseite des Verankerungsteiles eine Ringnut zur Aufnahme der Dichtungsscheibe bei der Montage vorgesehen wird. Dadurch kann der Injektionsspritzenkopf in fertig montiertem Zustand transportiert, sterilisiert und zwischengelagert werden, bevor er auf vorbereitete Spritzenzylinder-Kolbeneinheiten aufgesetzt wird.

Ein wesentlicher Vorteil der Erfindung besteht darin, daß das Medikament erst zum Zeitpunkt des Aktivierens der Fertigspritze, dem Abdrehen des Originalitätsverschlusses und Entfernen der Kanülenschutzkappe mit dem Inneren der Kanüle in Berührung kommt. Auf diese Weise wird ein Auskristallisieren des Medikamentes in der Kanüle, während der oft sehr langen Lagerzeit der Spritzen, und auch das damit verbundene Verstopfen der Kanüle vermieden. Bedingt durch die beschriebene Trennung zwischen dem Medikament und dem Fertigspritzen-Verschlußsystem ist die Sterilität auch über einen langen Zeitraum gewährleistet. Die Fertigspritze muß daher nach der Herstellung nicht zusätzlich keimfrei verpackt werden, sondern kann ohne weitere Überverpackung in Schachteln abgepackt werden.

Die Erfindung wird nachstehend an Hand eines Ausführungsbeispieles unter Bezugnahme auf die Zeichnungen näher erläutert, in denen zeigen:
Fig. 1 eine gesprengte Darstellung des erfindungsgemäßen Injektionsspritzenkopfes in Verbindung mit einem nur teilweise dargestellten Spritzenzylinder,
Fig. 2 im Axialschnitt einen erfindungsgemäßen Injektionsspritzenkopf,
Fig. 3 einen vergrößerten Schnitt nach der Linie 3-3 in Fig. 2;
Fig. 4 den Injektionsspritzenkopf von Fig. 1 in zusammengebautem Zustand im Axialschnitt,
Fig. 5 eine Darstellung des Injektionsspritzenkopfes nach Fig. 4 nach der Betätigung, und
Fig. 6 einen Axialschnitt eines weiteren Ausführungsbeispieles des Injektionsspritzenkopfes.

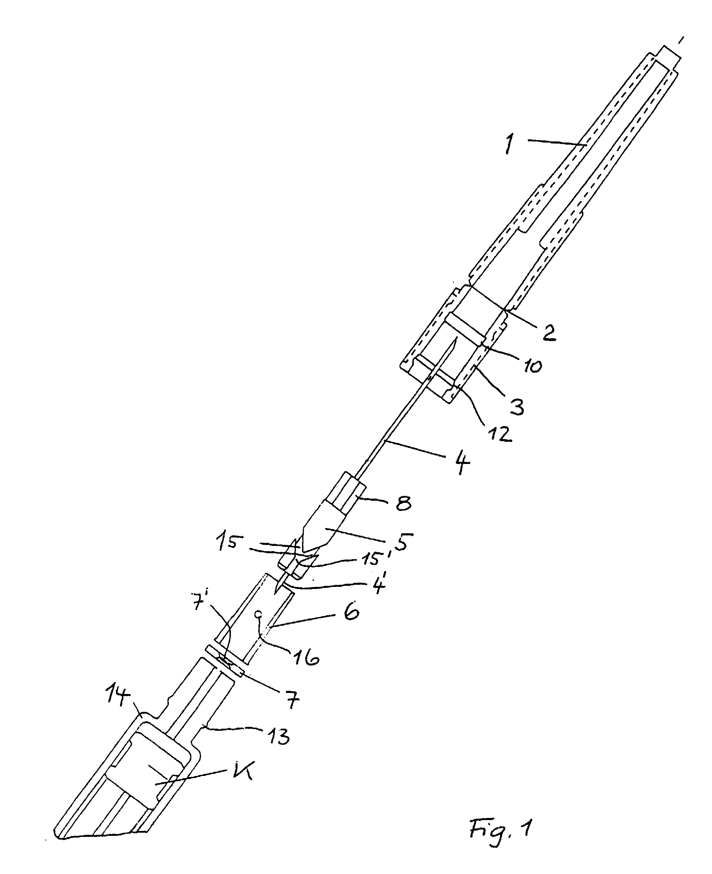

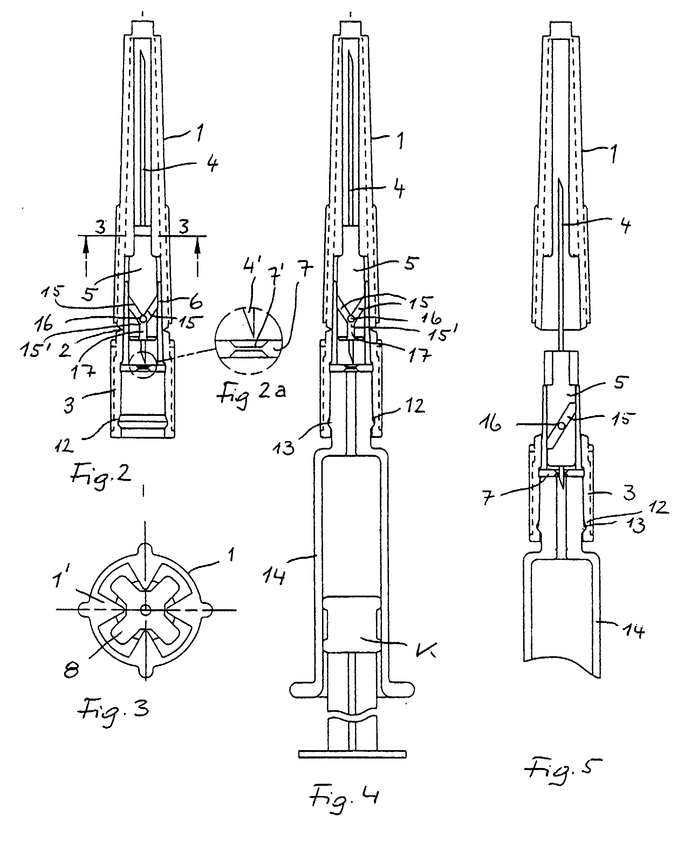

Der Injektionsspritzenkopf setzt sich gemäß den Fig. 1 und 2 im wesentlichen aus vier Montageteilen zusammen, u.zw. einer Schutzkappe 1, die über eine Sollbruchstelle 2 einstückig mit einem Verankerungsteil 3 verbunden ist, einer Injektionskanüle 4, die in einem Kanülenträger 5 fest verankert ist, z.B. durch Hochfrequenzverschweißen oder Verkleben, einer Kanülenträgerführung 6 und einer Dichtungsscheibe 7. Der Kanülenträger 5 ist, wie Fig. 3 zeigt, im Schnitt kreuzförmig mit vier Rippen 8 ausgebildet, zwischen welche radiale Rippen 1' der Kanülenschutzkappe 1 ragen.

Beim Zusammenbau des Injektionsspritzenkopfes wird der Kanülenträger 5 so weit in die Kanülenträgerführung 6 eingesetzt, bis das Ende 4' der Kanüle 4 aus der Kanülenträgerführung 6 auf der Seite der Dichtungsscheibe 7 vorsteht. Die Kanülenträgerführung 6 wird anschließend in den Verankerungsteil 3 und in die Schutzkappe 1 von deren freien Ende her eingeführt (Fig. 1), wobei durch eine konische Ausbildung des Außenumfanges der Kanülenträgerführung 6 und eine korrespondierende konische Ausbildung der Innenwand des Verankerungsteiles 3 ein zumindest kraftschlüssiger Preßsitz erzielt wird. Zum Abschluß wird die Dichtungsscheibe 7 in den Verankerungsteil 3 eingesetzt und rastet in einer Ringnut 10 am Innenumfang des Verankerungsteiles 3 ein. Der auf diese Weise fertiggestellte Injektionsspritzenkopf ist in Fig. 2 gezeigt und kann transportiert, sterilisiert und zwischengelagert werden, ohne daß die Gefahr einer Verunreinigung oder Beschädigung der Injektionskanüle 4 besteht.

Die in den Fig. 1 und 2 bzw. 2a dargestellte Dichtungsscheibe 7 bildet in ihrem zentralen Bereich eine Membrane 7'.

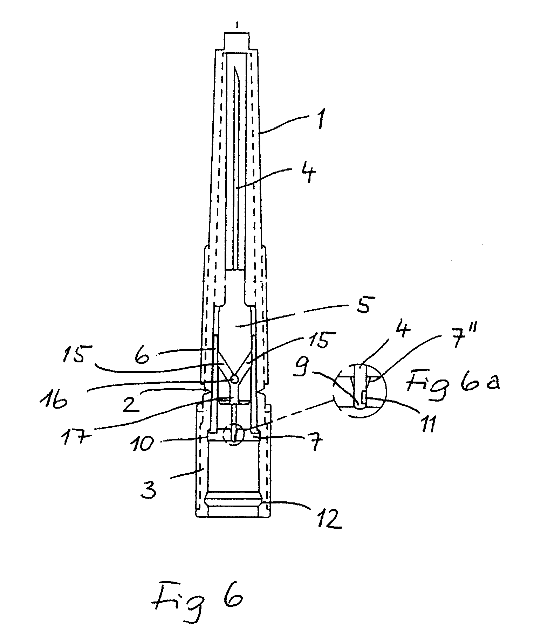

Das der Dichtungsscheibe 7 zugekehrte Ende 4' der Injektionskanüle 4 ist zugeschärft oder gemäß Fig. 6 bei 9 geschlossen und mit einer seitlichen Öffnung 11 versehen. Im ersten Fall wirkt die Injektionskanüle mit einer Dichtungsscheibe 7 zusammen, die in ihrem zentralen Bereich mit einer Membrane 7' versehen ist, welche von der Kanüle beim Aktivieren der Spritze durchstochen wird. Im Falle der Ausführungsform nach Fig. 6 ist die Dichtungsscheibe 7 vorgelocht und das geschlossene Ende der Kanüle verschließt das nach oben zu konisch erweiterte Loch 7" vor der Aktivierung der Spritze.

Der Verankerungsteil 3 ist aus elastischem Material gefertigt und mit einer Umfangsnut 12 auf einen Umfangswulst 13 des Zylinderhalses des Spritzenzylinders 14 aufschnappbar. Alternativ könnte der Verankerungsteil 3 auf den Zylinderhals des Spritzenzylinders 14 aufgepreßt oder durch Ultraschall- oder Hochfrequenzverformung angeformt werden. Der Spritzenzylinder 14 wird mit einem Arzneimittel gefüllt und durch Einsetzen einer Kolbeneinheit K abgedichtet. Die fertiggestellte Injektionsspritzenanordnung ist sodann zur Verwendung bereit.

Zur Einleitung des Injektionsvorganges wird der Kanülenträger 5 mit der Injektionskanüle 4 durch Abdrehen der Schutzkappe 1 in die Kanülenträgerführung 6 in beliebiger Richtung derart eingedreht, daß gemäß den Fig. 1 bis 5 die Dichtungsscheibe 7 durchstoßen wird bzw. gemäß Fig. 6 die Öffnung 11 der Kanüle 4 die Dichtungsscheibe 7 in ausreichendem Maße passiert hat, um in den Bereich des flüssigen Medikamentes zu gelangen. Für den dazu erforderlichen Drehantrieb des Kanülenträgers 5 ist die Schutzkappe 1 an ihrem Innenumfang mit den vier Rippen 1' versehen, welche in die Nuten des Kanülenträgers bzw. zwischen dessen Arme 8 eingreifen (Fig. 3). Durch Verdrehen der Schutzkappe 1 gegenüber dem Verankerungsteil 3 wird nach dem Durchbrechen der Sollbruchstelle 2 ein Drehantrieb des Kanülenträgers 5 und damit gemäß den Fig. 1 bis 5 ein Durchstoßen der Dichtungsscheibe 7 bewirkt. Die erreichte Stellung ist in Fig. 5 gezeigt. Bei der Ausführungsform nach Fig. 6 wird das mit der seitlichen Öffnung 11 versehene Ende 9 der Kanüle aus der in Fig. 6 gezeigten Stellung in den Spritzenzylinderraum hinein bewegt.

Der Kanülenträger 5 hat an seinem Umfang zwei Führungsnuten 15, die axialsymmetrisch mit vorbestimmter Steigung im wesentlichen V-förmig verlaufen. Die Führungsnuten 15 münden an ihrem der Dichtungsscheibe 7 zugekehrten Ende ineinander. In den der Dichtungsscheibe 7 zugekehrten Mündungsbereich 15' greift ein Führungszapfen 16 der Kanülenträgerführung 6 ein, der beim Zusammenstecken von Kanülenträger und Kanülenträgerführung über eine Einführnut 17 des Kanülenträgers, die sich in radialer Richtung gegen ihr Eintrittsende erweitert, in den Mündungsbereich 15 eingeführt wird. Eine Drehung der Schutzkappe 1 im Uhrzeigersinn oder Gegenuhrzeigersinn bewirkt auf diese Weise einen Durchtritt des Endes 4' der Injektionskanüle 4 durch die Dichtungsscheibe 7 bzw. einen Eintritt des Endes 9 der Kanüle in den Spritzenzylinder. Die Steigung der Führungsnuten 15 ist so gewählt, daß z.B. mit 1/4 Umdrehung des Kanülenträgers 5 ein Weg der Kanüle 4 relativ zur Dichtungsscheibe 7 von z.B. 3 mm erzielt wird. Dieser Weg reicht aus, damit die Kanülenschutzkappe 1 vom Verankerungsteil 3 abgedreht und die Dichtungsscheibe 7 sicher passiert wird, so daß das Medikament durch die zentrale Öffnung oder die seitliche Öffnung 11 der Kanüle 4 in den Kanülenhohlraum eintreten und injiziert werden kann.

Es versteht sich, daß die dargestellte Ausführungsform mit vier Rippen 1' bzw. Armen 8 nur beispielhaft ist. Es kann jede beliebige Anzahl von Rippen verwendet werden, welche einen drehfesten Eingriff zwischen Schutzkappe 1 und Kanülenträger 5 ermöglicht.

Die Schutzkappe 1 mit Verankerungsteil 3, der Kanülenträger 5 und die Kanülenträgerführung 6 sind bevorzugt spritzgußgeformte Kunststoffteile.

## Patentansprüche

1. Injektionsspritzenkopf mit Originalitätsverschluß für einen Spritzenzylinder (14), der einen Zylinderhals aufweist, welcher mit einem axialen Flüssigkeitsaustrittskanal und einem Umfangswulst (13) zur Verankerung des Injektionsspritzenkopfes versehen ist, mit einer Injektionskanüle (4), die in einem Kanülenträger (5) montiert ist, aus dem sie an beiden axialen Enden herausragt und der in einer Kanülenträgerführung (6) relativ zu dieser bewegbar angeordnet ist, und einer Kanülenschutzkappe (1), an die über eine Sollbruchstelle ein die Kanülenträgerführung (6) festhaltender, am Umfangswulst (12) des Zylinderhalses unter elastischer Verformung festlegbarer Verankerungsteil (3) mit einer Dichtungsscheibe anschließt, wobei die Kanülenschutzkappe (1) am Innenumfang in Axialrichtung verlaufende Rippen (1') aufweist, welche mit entsprechenden Rippen (8) des Kanülenträgers (5) für einen Drehantrieb desselben in Eingriff stehen, wobei am Umfang des Kanülenträgers (5) zwei axialsymmetrisch, mit vorbestimmter Steigung im wesentlichen V-förmig verlaufende und an ihrem der Dichtungsscheibe (7) zugekehrten Ende ineinandermündendc Führungsnuten (15) für einen im Inneren der Kanülenträgerführung (6) vorgesehenen Führungszapfen (16) ausgebildet sind, der im unbetätigten Zustand des Injektionsspritzenkopfes in den der Dichtungsscheibe (7) zugekehrten Mündungsbereich (15') der Führungsnuten (15) eingreift und bei einer Drehung der Kanülenschutzkappe (1) in jeder der beiden Drehrichtungen unter Zerstörung der Sollbruchstelle (2) relativ zu einer der beiden Führungsnuten gleitet, so daß der Kanülenträger gedreht und mit der Kanüle (4) relativ zur Dichtungsscheibe (7) bewegt wird, wobei an den der Dichtungsscheibe (7) zugekehrten Mündungsbereich (15') der Führungsnuten (15) eine nach unten offene axiale Einführnut (17) für den Führungszapfen (16) anschließt, die sich in radialer Richtung gegen ihr Eintrittsende zu erweitert, wobei das der Dichtungsscheibe (7) zugekehrte Ende (9) der Injektionskanüle (4) geschlossen und mit einer seitlichen Öffnung (11) versehen ist, durch welche das Medikament nach dem Aktivieren des Spritzenkopfes in die Kanüle eintreten kann, und wobei die Dichtungsscheibe (7') vorgelocht ist und die Injektionskanüle (4) das Loch im unbetätigten Zustand des Injektionsspritzenkopfes verschließt.

2. Injektionsspritzenkopf nach Anspruch 1, dadurch gekennzeichnet, daß die Steigung der Führungsnuten (15) so getroffen ist, daß eine 1/4 Umdrehung des Kanülenträgers (5) einen Weg der Kanüle (4) relativ zur Dichtungsscheibe (7) von 3 mm bewirkt.

3. Injektionsspritzenkopf nach Anspruch 1 oder 2, dadurch gekennzeichnet, daß der Verankerungsteil (3) innenseitig konisch ausgebildet und die Kanülenträgerführung (6) in den Verankerungsteil (3) von dessen offenen Ende her einpreßbar ist.

4. Injektionsspritzenkopf nach einem der Ansprüche 1 bis 3, dadurch gekennzeichnet, daß an der Innenseite des Verankerungsteiles (3) eine Ringnut (10) zur Aufnahme der Dichtungsscheibe (7, 7') vorgesehen ist.

## Claims

1. A hypodermic syringe head with a tamper-evident closure for a syringe cylinder (14), which has a cylinder neck that is provided with an axial fluid outlet conduit and a circumferential bead (13) for anchoring the hypodermic syringe head, having an injection cannula (4), which is mounted in a cannula carrier (5) from which it protrudes on both axial ends and which is disposed in a cannula carrier guide (6) so as to be movable relative thereto, and a cannula guard cap (1) which is adjoined, via a rated breaking point, to an anchoring element (3) with a sealing disk, which anchoring element holds the cannula carrier guide (6) and can be affixed with elastic deformation to the circumferential bead (12) of the cylinder neck, wherein the cannula guard cap (1) has axially extending ribs (1') on its inside circumference which engage corresponding ribs (8) of the cannula carrier (5) for rotationally driving it, wherein on the circumference of the cannula carrier (5) two axially symmetrical guide grooves (15) are formed for a guide peg (16), provided in the interior of the cannula carrier guide (6), which extend with a predetermined inclination substantially in a V and open into one another on their end oriented toward the sealing disk (7), and the guide peg, in the unactuated state of the hypodermic syringe head, engages the orifice region (15'), oriented toward the sealing disk (7), of the guide grooves (15) and upon a rotation of the cannula guard cap (1) in each of the two directions of rotation slides relative to one of the two guide grooves, destroying the rated breaking point (2), so that the cannula carrier is rotated and moved together with the cannula (4) relative to the sealing disk (7), wherein an axial introduction groove (17), open at the bottom, for the guide peg (16) adjoins the orifice region (15'), oriented toward the sealing disk (7), of the guide grooves (15) and widens in the radial direction towards its inlet end, wherein the end (9) of the injection cannula (4) oriented toward the sealing disk (7) is closed and is provided with a lateral opening (11), through which the medication can enter the cannula, after the syringe head has been activated, and wherein the sealing disk (7') is pre-perforated, and in the unactuated state of the hypodermic syringe head the injection cannula (4) closes the hole.

2. The hypodermic syringe head of claim 1, characterized in that the inclination of the guide grooves (15) is selected such that an approximately 1/4 rotation of the cannula carrier (5) causes a travel of approximately 3 mm on the part of the cannula (4) relative to the sealing disk (7).

3. The hypodermic syringe head of claim 1 or 2, characterized in that the anchoring element (3) is formed conically on the inside, and the cannula carrier guide (6) can be press-fitted into the anchoring part (3) from the open end thereof.

4. The hypodermic syringe head of one of claims 1-3, characterized in that an annular groove (10) for receiving the sealing disk (7, 7') is provided on the inside of the anchoring element (3).

## Revendications

1. Tête de seringue d'injection comportant un dispositif de fermeture de garantie pour un cylindre de seringue (14), qui comporte un col, qui est équipé d'un canal axial de sortie de liquide et d'un rebord circonférentiel (13) pour l'ancrage de la tête de la seringue d'injection, et comportant une canule d'injection (4), qui est montée dans un porte-canule (5), à partir de laquelle elle fait saillie sur les deux extrémités axiales et qui est disposée de manière à être déplaçable dans un guide (6) de porte-canule par rapport à ce dernier, et un capuchon de protection de canule (1), auquel se raccorde, par l'intermédiaire d'une zone de rupture de consigne, une partie d'ancrage (3) qui maintient fermement le guide (6) du porte-canule, peut être fixée sur le rebord circonférentiel (12) du col du cylindre moyennant une déformation élastique et comporte une rondelle d'étanchéité, le capuchon de protection de canule (1) possédant, sur sa périphérie intérieure, des nervures (1') qui s'étendent dans la direction axiale et qui engrènent avec des nervures correspondantes (8) du porte-canule (5) pour réaliser l'entraînement en rotation de ce dernier, et dans lequel sur la périphérie du porte-canule (5) sont formées deux rainures de guidage (15) qui s'étendent essentiellement en forme de V avec une dissymétrie axiale et avec une pente prédéterminée et se rejoignent au niveau de leur extrémité tournée vers la rondelle d'étanchéité (16) et sont conçues pour des tétons de guidage (16) prévus à l'intérieur du guide (6) du porte-canule, et qui, lorsque la tête de la seringue d'injection n'est pas actionnée, s'engage dans la zone d'embouchure (15') des rainures de guidage (15), qui est tournée vers la rondelle d'étanchéité (16), et, lors d'une rotation du capuchon (1) de protection de canule, glisse dans chacun des deux sens de rotation, en détruisant la zone de rupture de consigne (2), par rapport à l'une des deux rainures de guidage de sorte que le porte-canule tourne et est déplacé, avec la canule (4), par rapport à la rondelle d'étanchéité (16), auquel cas au niveau de la zone d'embouchure (15'), tournée vers la rondelle d'étanchéité (16), des rainures de guidage (15) se raccorde une rainure axiale d'introduction (17), ouverte vers le bas et prévue pour le téton de guidage (7) et qui s'élargit dans la direction radiale par rapport à son extrémité d'entrée, et dans lequel l'extrémité (9), tournée vers la rondelle d'étanchéité (7), de la canule d'injection (4) est fermée et est pourvue d'une ouverture latérale (11) par laquelle le médicament peut pénétrer dans la canule après activation de la tête de la seringue, et dans lequel la rondelle d'étanchéité (7') est préalablement perforée et la canule d'injection (4) ferme le trou lorsque la tête de la seringue d'injection est dans l'état non actionné.

2. Tête de seringue d'injection selon la revendication 1, caractérisée en ce que la pente des rainures de guidage (15) est choisie de telle sorte que 1/4 de rotation du porte-canule (5) provoque un déplacement de la canule (4) de 3 mm par rapport à la rondelle d'étanchéité (7).

3. Tête de seringue d'injection selon la revendication 1 ou 2, caractérisée en ce que la partie d'ancrage (3) est agencée avec une forme conique sur son côté intérieur et que le guide (6) du porte-canule peut être enfoncé dans la partie d'ancrage (3) à partir de son extrémité ouverte.

4. Tête de seringue d'injection selon l'une des revendications 1 à 3, caractérisée en ce qu'une gorge annulaire (10) servant à recevoir la rondelle d'étanchéité (7,7') est prévu sur la face intérieure de la partie d'ancrage (3).
